Europäisches Patentamt

European Patent Office

Office européen des brevets

(19)

(11) Publication number: **0 439 338 A1**

(12) **EUROPEAN PATENT APPLICATION**

(21) Application number: **91300508.8**

(22) Date of filing: **23.01.91**

(51) Int. Cl.⁵: **C07C 17/20, B01J 31/02, B01J 27/08, B01J 37/22**

(30) Priority: **25.01.90 GB 9001762**

(43) Date of publication of application:
**31.07.91 Bulletin 91/31**

(84) Designated Contracting States:
**BE CH DE FR GB IT LI NL SE**

(71) Applicant: **IMPERIAL CHEMICAL INDUSTRIES PLC**
**Imperial Chemical House, Millbank**
**London SW1P 3JF (GB)**

(72) Inventor: **Thomson, James**
**11 Pine Court**
**Abronhill, Cumbernauld G67 3AY (GB)**
Inventor: **Winfield, John Michael**
**133 Essex Drive**
**Glasgow G14 9PD (GB)**
Inventor: **Webb, Geoffrey**
**39 Pelstream Avenue**
**Stirling FK7 0BG (GB)**

(74) Representative: **Oldroyd, Alan et al**
**Imperial Chemical Industries PLC Legal**
**Department: Patents PO Box 6 Bessemer**
**Road**
**Welwyn Garden City Herts, AL7 1HD (GB)**

(54) A catalyst for halogen exchange in halohydrocarbons and for acid/base reactions.

(57) A catalyst for halogen exchange in halohydrocarbons or for acid/base reactions comprising a halogenated gamma-alumina having a hydrocarbonaceous or halohydrocarbonaceous material laid down thereon. The catalyst is prepared by halogenating gamma-alumina with $SF_4$, $SOF_2$, $COF_2$ $COCl_2$ or $CCl_4$ and conditioning the halogenated alumina with a gaseous olefin or a halohydrocarbon to lay down a hydrocarbonaceous or a halohydrocarbonaceous material on its surface. The catalyst is useful for producing fluorohydrocarbons or chlorofluorohydrocarbons by reacting HF with halohydrocarbons under mild conditions.

EP 0 439 338 A1

# A CATALYST FOR HALOGEN EXCHANGE IN HALOHYDROCARBONS AND FOR ACID/BASE REACTIONS

The present invention relates to a novel catalyst, its production and its use for halogen exchange in halohydrocarbons and for general acid/base reactions under relatively mild reaction conditions.

Chlorofluorocarbons (CFCs) are widely used as refrigerants, aerosol propellants, solvents and blowing agents. Certain of the simpler CFCs have in recent years been implicated in damage to the atmospheric ozone layer ; the inert CFCs are believed to diffuse up through the atmosphere until they are decomposed by ultraviolet radiation with the production of chlorine atoms which can initiate catalytic ozone destruction chains. There is a need to replace these environmentally damaging substances, whose use is controlled by the Montreal Protocol, and to replace them with more acceptable materials containing hydrogen atoms. Possible candidates include $CH_3CF_3$ (143a), $CH_2FCF_3$ (134a), $CH_3CF_2Cl$ (142b) and $CH_3CFCl_2$ (141b).

Conventionally CFCs are produced by fluorination of chloro(hydro)carbons with HF at elevated temperatures of 350°-450°C in the vapour phase using a fluorinated chromia catalyst. Japanese Patent Application 79/108420 filed 24th August, 1979 (Chemical Abstracts 95 :97010a) describes the use of aluminium trifluoride supported chromia treated with 1,2-dichlorotetrafluoroethane as catalyst for the fluorination of chloroalkenes at 350°C. However, lower temperature processes have been proposed and German Published Patent Specification DE 3603103 discloses the preparation of chlorofluorohydrocarbons by the fluorination of chlorohydrocarbons using a catalyst $MgFe_a(Cu$ and/or $Mn)_bX_c$ (where $X = 0$, OH or anions from acids volatile at <200°C; $a = 3 \times 10^{-3}$ to 1.5 ; $b = 0$ to 2.6 and c has a value to satisfy the cation valency required such that $0 < b/a < 210$) at 160°C.

The halogenation of gamma-alumina is well documented (see eg Catalysis Reviews (1971) 5, 67 ; Ind. Eng. Chem. Process Res. Dev. 1977, 16, 12 ; and United States Patent No 4,427,791.) and particularly the incorporation of chlorine and fluorine to increase catalyst acidity in the context of bifunctional reforming catalysis. However, the use of such halogenated gamma-alumina surfaces for the production of catalytically active organic overlayers has not hitherto been reported.

High temperature fluorination processes pose significant production problems and it is an object of the present invention to allow the use of milder reaction conditions. Further objects of the present invention are to provide a catalyst for the preparation of CFC alternatives under mild reaction conditions and to provide a method for the production of supported organic layer cataysts for use in acid/base catalysed reactions.

A first aspect of the present invention provides a catalyst for halogen exchange in halohydrocarbons and/or for acid/base catalysed reactions, which comprises gamma-alumina containing a source of halogen and having hydrocarbonaceous or halohydrocarbonaceous material laid down thereon.

The material laid down on the halogenated gamma-alumina should not be carbon itself ; it is a (halo)hydrocarbonaceous material which may be, for example, a complex oligomeric material of overall general formula $(CH_nX_m)_x$

where $(m + n)$ is less than or equal to 2, and is preferably in the range 1.0 to 1.8 (particularly 1.2 to 1.5);

X represents a possible substituent such as halogen ; and

x represents the degree of polymerisation of the monomer from which the material is derived (for example 2 to 25).

The (halo)hydrocarbonaceous material may be bonded by ionic or covalent bonds to the gamma-alumina surface at a number of positions. Furthermore, the material is usually a mixture of different species having different chemical structures or degrees of polymerisation, such as possibly halogenated polyacetylenes and polyolefins.

A second aspect of the present invention provides a process for the preparation of the catalyst, which comprises two sequential, but equally important steps ;

(i) reacting calcined gamma-alumina with a halogenating agent selected from $SF_4, SOF_2 COF_2$, $COCl_2$, and $CCl_4$, to produce a halogenated gamma-alumina ; and

(ii) treating the halogenated gamma-alumina with a gaseous olefin or a halohydrocarbon so as to lay down (halo)hydrocarbonaceous material on the alumina surface.

Gamma-alumina is a well known catalyst substrate material. Calcination of gamma-alumina results in the removal of $H_2O$,-OH and bridging $H^+$ groups from the structure and the creation of surface Brønsted and Lewis acid sites. Calcination of gamma-alumina at 250°C is believed to remove about 40% of the -OH groups from the structure. Halogenation followed by further calcination or degassing results in the removal of further -OH groups and the formation of strongly acidic sites ; for example chlorination using $CCl_4$ and calcination at 250°C produces a surface with about 40% Lewis acid sites and about 60% Brønsted acid sites.

Halogenation of the calcined alumina using $SF_4$ may be performed at ambient temperature, although with

$COCl_2$, $COF_2$, $SOF_2$ or $CCl_4$, higher halogenation temperatures, typically 250°C, are required.

The relative proportions of reagents used in the halogenation step of the catalyst preparation are chosen to avoid the production of $AlX_3$ (X = halogen) phases. For example, an incorporation of 2-7 mg atoms of halogen per gram of gamma-alumina is preferred.

The second stage in the catalyst preparation treatment, which is an essential step in the production of the active catalyst, is carried out by treatment of the halogenated gamma-alumina with a gaseous olefin or with a halogenated hydrocarbon so as to deposit a (halo)hydrocarbonaceous layer on the surface of the gamma—alumina. $C_2$ and higher olefins, including ethenes, propenes, butenes and butadienes and $C_2$ and higher halogenated hydrocarbons are suitable for this treatment, the temperature being chosen to avoid complete cracking of the gaseous olefin or halogenated hydrocarbon and the resultant laying down of carbon on the alumina surface. Temperatures in the range 20°-250°C can be used, preferably up to 150°C.

A molar ratio of halogen to olefin or halohydrocabon treating agent of at least 1 :1 is preferred.

A third aspect of the invention provides use of the catalyst for the production of fluorohydrocarbons and halofluorohydrocarbons by treating a halohydrocarbon containing at least one halogen other than fluorine with anhydrous HF in the presence of the catalyst.

The halohydrocarbon starting material is generally a chloro, bromo or iodo $C_2$-$C_6$ halohydrocarbon. Since the presence of fully fluorinated groups tends to inhibit further fluorination reactions, it has been found desirable to keep the HF concentration as low as possible commensurate with an acceptable rate of reaction in order to avoid or delay the formation of the fully- fluorinated groups. Generally, the molar ratio of the halohydrocarbon to HF is at least 1 :1.

It is a particular advantage of the method that it may often proceed at a satisfactory rate at relatively low temperatures, eg in the range subambient to 150°C (particularly 20° to 100°C). In general, the activity of the catalyst towards the fluorination of chlorohydrocarbons decreases as the reaction temperature increases and it is preferred to keep the reaction temperature below about 75°C, at which temperature the hydrocarbonaceous material of the catalyst appears to lose stability and starts to break down.

A fourth aspect of the invention provides a method of performing acid/base catalysed reactions, which comprise reaction of suitable organic substrates over the aforementioned catalyst. Thus the catalyst is useful for acid/base reactions which proceed via carbonium or carbanion intermediates, such as Friedel-Craft's reactions skeletal rearrangement, isomerisation and oligomerisation. For example, the catalyst can be used for isomerisation of olefins such as butenes and for the alkylation of olefins using simple (eg $C_{1-6}$ especially $C_1$) alkyl halides. The catalyst can be used for these reactions under relatively mild conditions and is suitable for use in a continuous production process.

Embodiments of the invention will now be described by way of example only.

EXAMPLE 1

This example describes the preparation of a catalyst using $SF_4$ as the halogenating agent and 1,1,1-trichloroethane as the conditioning agent.

Gamma-alumina (0.5g) calcined at 250°C was reacted at ambient temperature with $SF_4$ (4mmol) to produce a fluorinated gamma-alumina. In this process the $SF_4$ was hydrolysed to $SO_2$, $SOF_2$ and HF. $^{18}$F-fluorine labelled $SF_4$ studies showed that, after a reaction time of one hour the fluorine content of the treated gamma-alumina was 3.5 ± 0.2 mg atom fluorine per gram of catalyst.

In order to quench the cracking properties of the $SF_4$-treated gamma-alumina surface, 1,1,1-trichloroethane (3 mmol) was allowed to react at room temperature with the fluorinated alumina (0.5g) for 4 hr. Hydrocarbonaceous material was laid down on the fluorinated gamma-alumina surface as shown by a purple colouration. The $^{19}$F n.m.r. spectrum of the gaseous products revealed a range of halocarbons (including $CFCl_2CH_3$, $CF_2ClCH_3$, $CF_3CH_3$, $CCl_3CH_3$ and $CH_2 = CCl_2$) without any cracking of the 1,1,1-trichloroethane, ie no $C_1$ products were observed.

EXAMPLE 2

This example describes the preparation of a catalyst using $CCl_4$ as the halogenating agent and but-1-ene as the conditioning agent.

Gamma-alumina (ca. 0.6g) calcined at 250°C was halogenated using $CCl_4$ (2 mmol) at 225°C for 6hr to produce chlorinated gamma-alumina. In this process the $CCl_4$ was hydrolysed to $CO_2$, $COCl_2$ and HCl. The chlorine content of the gamma-alumina, as determined by neutron activation analysis, was 2.25 ± 0.003 mg atom chlorine (g alumina)$^{-1}$. But-1-ene was reacted over the treated surface in a Monel pressure vessel at room temperature for 3hr. Subsequent chromatographic analysis of the gas phase revealed only but-1-ene. The catalyst showed a deep yellow colouration due to hydrocarbonaceous material laid down on its surface.

EXAMPLE 3

This example describes the preparation of a catalyst using $COF_2$ as the halogenating agent and but-1-ene as the conditioning agent.

Gamma-alumina (ca. 0.5g) was calcined at 250°C and then treated with $COF_2$ (3 mmol) at 230°C for 6hr. The $COF_2$ was hydrolysed, producing $CO_2$ and fluorinated gamma_alumina. But-1-ene (3 mmol) was reacted over the fluorinated alumina at room temperature for 3hr in a Monel pressure vessel. Analysis of the gaseous reaction products showed that the but-1-ene had isomerised to cis- and trans-but-2-ene. The catalyst had become covered with a layer of hydrocarbonaceous material.

EXAMPLE 4

This example demonstrates the production of chlorofluorohydrocarbons (HCFCs) by the fluorination of 1,1,1-trichloroethane with HF using a catalyst prepared as described in Example 1.

A series of five catalytic experiments was carried our using a sample of the catalyst (0.54g) as produced in Example 1 and having fluorine content of ca. 1.65mg atom fluorine, and $CH_3CCl_3$ (3 mmol) and HF (1 mmol) mixtures. All reactions were carried out in a Monel metal pressure vessel (volume = 95 cm³) at ambient temperature for 2 hours. Product analyses by $^1$H and $^{19}$F n.m.r. spectroscopy gave the product distributions shown in Tables 1 and 2. HCl was also identified as a reaction product.

The catalytic reaction was performed five times using the same sample of catalyst. The extent of conversion of $CH_3CCl_3$ to HCFCs was not determined, but the theoretical value based on total consumption of the gaseous HF is expected to be 33.3%. The formation of $CF_3CH_3$ and $CF_2ClCH_3$ was not favoured compared with $CFCl_2CH_3$.

Table 1 : Organic products from the treatment and catalytic reactions (determined by $^1$H n.m.r. analysis of the liquid mixtures).

| Reaction | Mole ratio A | B | C | D | E |
|---|---|---|---|---|---|
| Treatment Step | 92.5 | 4.0 | 2.5 | 0.75 | 0.2 |
| Catalytic | | | | | |
| Run 1 | 76.9 | 4.5 | 7.2 | 7.6 | 3.6 |
| Run 2 | 68.7 | 5.3 | 24.3 | 1.4 | 0.35 |
| Run 3 | 76.3 | 0.5 | 22.5 | 0.75 | trace |
| Run 4 | 85.0 | 0.3 | 12.7 | 1.1 | 1.0 |
| Run 5 | 95.0 | trace | 4.0 | 0.75 | 0.25 |

A=1,1,1-trichloroethane $CCl_3CH_3$
B=1,1-dichloroethene $CH_2=CCl_2$
C=1-fluoro 1,1-dichloroethane $CFCl_2CH_3$
D=1,1-difluoro 1-chloroethane $CF_2ClCH_3$
E=1,1,1-trifluoroethane $CF_3CH_3$

Table 2 : HCFCs identified from $^{19}$F n.m.r. examination of the volatile products.

| Reaction | Mole ratio | C | D | E |
|---|---|---|---|---|
| Treatment Step | | 1.00 | 0.48 | 0.17 |
| Catalytic Run 1 | | 1.00 | 1.22 | 0.61 |
| Run 2 | | 1.00 | 0.08 | 0.02 |
| Run 3 | | 1.00 | 0.03 | 0.004 |
| Run 4 | | 1.00 | 0.10 | 0.02 |
| Run 5 | | 1.00 | 0.06 | 0.008 |

A further series of experiments using $CH_3CCl_3$ :HF molar ratios of 1 :2 and 1 :3 were less successful in producing HCFCs than those using a molar ratio of 3 :1.

EXAMPLE 5

Gamma-alumina (Degussa 'C' grade) (0.5g) was calcined at 250°C in a Monel metal bomb. $SF_4$ (3 mmol) was then condensed into the bomb at 77°K and the bomb was sealed and allowed to warm up to room temperature. The contents of the bomb were allowed to react at room temperature for 2 hours after which time the bomb was degassed by pumping. The gases were analysed by gas infra red spectroscopy and the procedure was repeated until $SF_4$ appeared in the gases withdrawn from the bomb.

Dry 1,1,1-trichloroethane (3 mmol) was condensed onto the fluorinated alumina at 77°K and the bomb was sealed and allowed to warm up to room temperature. After 4 hours at room temperature, the bomb was degassed.

A mixture of anhydrous HF (1 mmol) and $CCl_4$ (3 mmol) was condensed into the bomb at 77°K and the bomb was sealed and allowed to warm up to room temperature. After two hours at room temperature the bomb degassed and the gas-phase products were condensed in vacuo over NaF and analysed by $^1$H and $^{19}$F n.m.r spectroscopy.

The results showed the production of 1,1,2-trichloro-1,2,2-trifluoroethane (CFC-113) together with 1,1,1-trichloroethane.

EXAMPLE 6

The procedure described in Example 5 was repeated except that tetrachloroethylene, $C_2Cl_4$ was used instead of $CCl_4$.

The results showed the production of 1,1,1-trichloro-2,2,2-trifluoroethane (HCFC-133a) together with 1,1,1-trichloroethane, 1,1-dichloroethylene and 1,1,2-trichloroethylene.

EXAMPLE 7

The procedure described in Example 5 was repeated except that 1,1,2-trichloro-1,2,2-trifluoroethane (CFC 113) was used instead of $CCl_4$ and the fluorination reaction proceeded for 4 hours rather than 2 hours.

The following products were identified :
1,1-dichloro-1-fluoroethane (HCFC 141b)
1-chloro-1,1-difluoroethane (HCFC 142b)
1,1-dichloroethylene
1,1,1-trichloroethane

## EXAMPLE 8

The procedure described in Example 5 was repeated using 1,1,1-trichloro-2,2,2-trifluoroethane (CFC 113a) instead of CCl$_4$ and effecting reaction for 4 hours instead of 2 hours.

The following products were identified :

1,1-dichloro-1-fluoroethane (HCFC 141b)

1,1,2-trichloro-1,2,2-trifluoroethane (CFC 113)

1-chloro-1,1-difluoroethane (HCFC 142b)

1,1-dichloroethylene

1,1,1-trichloroethane

## EXAMPLE 9

The procedure described in example 5 was repeated using various starting materials (see below) instead of CCl$_4$. The following products were identified :

Run 1 - chloroform (CHCl$_3$)

Products :

1,1,2_trichloro-1,2,2-trifluoroethane (CFC 113)

1,1,1-trichloroethane

Run 2 - trichloroethylene

Products :

1,1,2-trichloro-1-fluoroethane (HCFC 131b) 10.1 mol %

1,2-dichloro-1,1-difluoroethane (HCFC 132b) 0.3 mol %

1,1-dichloro-1-fluoroethane (HCFC 141b) 1.18 mol %

1-chloro-1,1-difluoroethane (HCFC 142b) 1.24 mol %

1,1,1-trichloroethane 9.1 mol %

Run 3 - Asym-tetrachloroethane

Products :

1,1,2-trichloro-1-fluoroethane (HCFC 131b) 0.1 mol %

1,2-dichloro-1,1-difluoroethane (HCFC 132b) 4.3 mol %

1,1-dichloro-1-fluoroethane (HCFC 141b) 9.7 mol %

1-chloro-1,1-difluoroethane (HCFC 142b) 3.8 mol %

1,1,1-trifluoroethane (HCFC 143a) 1.1 mol %

Run 4 - Sym-tetrachloroethane

Products :

1,1-dichloro-1-fluoroethane (HCFC 141b) 20.4 mol %

1-chloro-1,1-difluoroethane (HCFC 142b) 4.4 mol %

1,1,1-trifluoroethane (HFC 143a) 2.7 mol %

1,2-dichlorotetrafluoroethane (CFC 114) 8.9 mol %

1,1,1-trichlorotrifluoroethane (CFC 113a) 1.7 mol %

1,1-dichloroethylene 16.12 mol %

Run 5 - 2,2-dichloropropane

Products :

2,2-chlorofluoropropane - 6.15 mol %
2,2-difluoropropane - 9.4 mol %
1,1,1-trichloroethane - trace

EXAMPLE 10

The procedure described in Example 5 was repeated except that the fluorinated alumina was conditioned with tetrachloroethylene for 3 hours instead of 1,1,1-trichloroethane for 2 hours.

Products :

1,1-dichloro-1-fluoroethane (HCFC 141b) 33.6 mol %
1-chloro-1,1-difluoroethane (HCFC 142b) 4.0 mol %
1,1,1-trifluoroethane (HFC 143a) trace
1,1-dichloroethylene 62.4 mol %

EXAMPLE 11

A catalyst was prepared as described in Example 5 except that the alumina halogenating agent was CCl$_4$ instead of SF$_4$ and halogenation was carried at 227°C instead of room temperature.
After conditioning the chlorinated alumina with 1,1,1-trichloroethane as described in Example 5, the resulting catalyst was used to react HF with 1,1,1-trichloroethane for 2 hours.

Products :

1,1-dichloro-1-fluoroethane (HCFC 141b) 1.3 mol %
1,2-dichlorotetrafluoroethane (CFC 114) 0.21 mol %
1,1,2-trichloro-1,2,2-trifluoroethane (CFC 113) trace
1,1-dichloroethylene 0.8 mol %

EXAMPLE 12

A catalyst was prepared as described in Example 5 except that conditioning agent was 2,2-dichloropropane and conditioning was for 3 hours instead of 2 hours.
The products from the conditioning step were 2,2-difluoropropane (7.02 mol%), 2-chloro-2-fluoropropane (2.67 mol%) and unreacted 2,2-dichloropropane (90.31 mol%).
The catalyst was used in the reaction of HF with 1,1,1-trichloroethane by the procedure described in Example 5.

Products :

1,1-dichloro-1-fluoroethane (HCFC 141b) - 1.6 mol%
1-chloro-1,1-difluoroehane (HCFC 142b) - 0.2 mol%
2,2-difluoropropane - 1.1 mol%
1,1-dichloroethylene - 2.4 mol%

EXAMPLE 13

The procedure described in Example 5 was repeated except that the catalyst conditioning agent was perchloroethylene instead of 1,1,1-trichloroethane. The product mixture was withdrawn and analysed and are shown below as Run 1.
The catalyst from Run 1 was re-used in a further reaction (Run 2) and then re-used in further Runs 3 to 6. The products of each Run were analysed and are shown below for each Run in Table 3.

Products: 1,1-dichloro-1-fluoroethane  }Unseparated
(HCFC 141b)  }mixture of
  }b.p. 32°C

1,1-dichloroethylene (DCE)  }
1-chloro-1,1-difluoroethane
(HCFC 142b)
1,1,1-trifluoroethane
(HFC 143a)
Unidentified species (UIS).

Table 3:

|  | Mol % Products | | | |
|  | 141b/DCE | 142b | 143a | UIS |
| Run 1 | 30.8 | 3.9 | trace | 12.9 |
| Run 2 | 39.5 | 4.3 | trace | – |
| Run 3 | 36.3 | 5.6 | trace | 9.7* |
| Run 4 | 6.4 | – | – | – |
| Run 5 | – | – | – | – |
| Run 6 | – | – | – | – |

* Retention time on column suggests
1,1,2-trichloro-1,2,2-trifluoroethane (CFC 113).

EXAMPLE 14

Using the procedure described in Example 5, 2,2-dichloropropane was fluorinated using a catalyst conditioned with 1,1,1-trichloroethane. The catalyst was used in 5 Runs and the products of each Run were determined and are shown in Table 4.

Products :

2-chloro-2-fluoropropane     (CFP)
2,2-difluoropropane          (DFP)
Unidentified species         (UIS)

Table 4:

| | Mol % Products | | |
|---|---|---|---|
| | CFP | DFP | UIS |
| Run 1 | 13.7 | 27.5 | - |
| Run 2 | 19.7 | 27.7 | - |
| Run 3 | 9.9 | 24.6 | - |
| Run 4 | - | 29.3 | 10.4 |
| Run 5 | - | - | - |

## Claims

1. A catalyst for halogen exchange in halohydrocarbons and/or for acid/base catalysed reactions which comprises gamma-alumina containing a source of halogen and having a hydrocarbonaceous or halohydrocarbonaceous material laid down thereon.

2. A catalyst as claimed in claim 1 wherein the material laid down thereon comprises a complex oligomeric material having the overall general formula $(CH_nX_m)_x$ where
   $(m + n)$ is less than or equal to 2,
   X is hydrogen or halogen, and
   x represents the degree of polymerisation of the compound from which the material is derived.

3. A catalyst as claimed in claim 2 wherein $(m + n)$ is in the range 1.0 to 1.8.

4. A catalyst as claimed in claim 3 wherein $(m + n)$ is in the range 1.2 to 1.5.

5. A catalyst as claimed in claim 2, 3 or 4 wherein X is chlorine or fluorine.

6. A catalyst as claimed in any one of claims 2 to 5 wherein x is from 2 to 25.

7. A catalyst as claimed in any one of the preceding claims wherein the material laid down thereon is derived from a gaseous olefin.

8. A catalyst as claimed in any one of claims 1 to 6 wherein the material laid down thereon is derived from a halohydrocarbon.

9. A process for the preparation of a catalyst which comprises the steps of
   (i) reacting calcined gamma-alumina with a halogenating agent selected from $SF_4$, $SOF_2$, $COF_2$, $COCl_2$ and $CCl_4$ to produce a halogenated gamma-alumina, and
   (ii) treating the halogenated gamma-alumina with a gaseous olefin or a halohydrocarbon so as to lay down a hydrocarbonaceous or halohydrocarbonaceous material on the alumina surface.

10. A process as claimed in claim 9 wherein the treatment step (ii) is carried out at a temperature in the range 20°C to 250°C.

11. A process as claimed in claim 9 or 10 wherein in step (ii) the ratio of halogen in the halogenated alumina to the olefin or halogenated hydrocarbon is at least 1 :1.

12. A process as claimed in claim 9, 10 or 11 wherein in step (i) 2 to 7 mg atom of halogen is incorporated per gm of the gamma-alumina.

13. A process for the production of fluorohydrocarbons and halofluorohydrocarbons which comprises treating a halohydrocarbon containing at least one halogen other than fluorine with anhydrous HF in the presence of a catalyst as claimed in claim 1.

14. A process as claimed in claim 13 wherein the molar ratio of the halohydrocarbon to HF is at least 1 :1.

15. A process as claimed in claim 13 or 14 wherein the temperature is no greater than 150°C.

16. A process as claimed in claim 13, 14 or 15 wherein the halohydrocarbon contains at least three halogen atoms.

17. A method of performing acid/base catalysed reactions which comprises reacting organic substrates over a catalyst as claimed in claim 1.

European Patent
Office

**EUROPEAN SEARCH REPORT**

Application Number

EP 91 30 0508

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 465 786 (H.F. ZIMMER)<br>* Claims 1,4; example 1 *<br>--- | 1,9,13,<br>15,16 | C 07 C 17/20<br>B 01 J 31/02<br>B 01 J 27/08<br>B 01 J 37/22 |
| A | GB-A- 770 640 (FARBWERKE HOECHST)<br>--- | | |
| A | EP-A-0 226 492 (ATOCHEM)<br>* Claim 8 *<br>----- | | |

TECHNICAL FIELDS
SEARCHED (Int. Cl.5)

C 07 C
B 01 J

The present search report has been drawn up for all claims

| Place of search | Date of completion of the search | Examiner |
|---|---|---|
| THE HAGUE | 08-05-1991 | THION M.A. |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons

& : member of the same patent family, corresponding
 document

EPO FORM 1503 03.82 (P0401)